# EUROPÄISCHE PATENTANMELDUNG

(11) **EP 2 277 592 A1**
(43) Veröffentlichungstag der Anmeldung: **26.01.2011**
(21) Anmeldenummer: 09166266.8
(22) Anmeldetag: 23.07.2009
(51) Int. Cl.: A61N 5/10

(54) **Verfahren und Einrichtung zur Strahlungssimulation eines zu kontrollierenden Auftreffens eines zu richtenden Strahlsenders auf ein Zielvolumen**

(71) Anmelder: Siemens Schweiz AG, 8047 Zürich (CH)
(72) Erfinder: Broennimann, Thomas, 4704, Wolfisberg (CH)
(74) Vertreter: Fischer, Michael

(57) **Zusammenfassung**

Es werden ein Verfahren und ferner eine Einrichtung zur Strahlungssimulation eines zu kontrollierenden Auftreffens eines ersten, zu richtenden Strahlsenders auf ein Zielvolumen eines Gegenstandes vorgeschlagen, für welche:
- mindestens ein dem ersten, stillgelegten Strahlsender räumlich gekoppelten Ultraschallsender auf das Zielvolumen unter einer orientierbaren Einstrahlrichtung gerichtet wird;
- mindestens ein ultraschall-basierter Schuss aus dem Ultraschallsender eingeleitet wird, welcher im Zielvolumen lokal und punktförmig auftrifft,
- mindestens eine Ultraschallsonde mindestens einen Anteil des Zielvolumen in einem Ultraschallbild aufnimmt,
- ein Mittel zur Ermittlung der Position einer ultraschallbasierten Markierung des Schusses in dem Bild verwendet wird,
- ein Abstand zwischen der Position der Markierung und einer Lage des zu kontrollierenden Auftreffens ermittelt und überprüft wird.

## Beschreibung

Die Erfindung betrifft ein Verfahren und eine Einrichtung zur Strahlungssimulation eines zu kontrollierenden Auftreffens eines zu richtenden Strahlsenders auf ein Zielvolumen nach dem Oberbegriff der Ansprüche 1 und 5.

Als Strahlsender ist jede Art von Strahler oder dessen Quelle vorgesehen. Die Erfindung fokussiert insbesondere auf radiotherapeutische Strahlsender oder technologische Äquivalente, welche meistens derart verwendet werden, um ungesunde organische Zelle in-vivo zu zerstören. Bei solchen Methoden ist es daher wichtig, eine genauere Bestrahlung zu gewährleisten, damit alle ungesunden Zellen vom zu richtenden Strahlsender innerhalb eines Zielvolumens (wie eines Tumors oder eines Karzinoms im Innenkörper eines Patienten) gezielt und aufgetroffen werden können, ohne dass gesunde Zellen getroffen werden.

Derzeit sind zahlreiche Methoden verwendet, um einen derartigen Tumor vor sowie während einer Bestrahlungstherapie zu lokalisieren. Insbesondere effektiv ist eine Röntgen-Methode, zB um eine Simulation vor der tatsächlichen Bestrahlung mittels eines Linearbeschleunigers durchzuführen. Dabei ist der Röntger-Simulator ein eigenständiges Gerät, welches an der Abbildung der Strahlungsgeometrie des Linearbeschleunigers genau angepasst ist und dessen Röntgenröhren- und Führung den hochenergetischen Strahls des Linearbeschleunigers ersetzen und simulieren kann. Das Auftreffen der Röntgen-Strahlung im Zielvolumen wird auf einem Film oder mittels Bildverstärkerröhre bildlich wiedergegeben. Anhand der Bilder welche von verschiedenen Stativwinkeln geschossen werden, kann die räumliche Präzision der späteren Bestrahlung mit dem Linearbeschleuniger erhöht werden.

Ein derartiger Röntgen-Simulator macht jedoch Gebrauch von Röntgenstrahlungen, welche für bereits erkrankte und ferner zu bestrahlenden Patienten etwa schädigend und mühsam sind.

Ein Ziel der vorliegenden Erfindung besteht daher darin, eine möglichst nicht schädliche Methode sowie eine entsprechende Einrichtung zu finden, mit welchen eine Strahlungssimulation eines zu kontrollierenden Auftreffens eines zu richtenden Strahlsenders auf ein Zielvolumen gewährleistet wird. Das Zielvolumen kann z.B. ein Tumor oder eine andere krankhafte oder zu behandelnde Stelle im Inneren Körper eines Patienten sein.

Erfindungsgemäß wird die Aufgabe durch die Merkmale der Ansprüche 1 und 5 gelöst. Ferner werden auch Ansprüche für die vorteilhafte Verwendungen der Einrichtung dargestellt. Vorteilhafte Ausgestaltungen der Erfindung sind in den Unteransprüchen dargelegt.

Dabei wird zuerst ein Verfahren zur Strahlungssimulation eines zu kontrollierenden Auftreffens eines ersten, zu richtenden Strahlsenders auf ein Zielvolumen eines Gegenstandes vorgeschlagen, für welches:
- mindestens ein dem ersten, stillgelegten Strahlsender räumlich gekoppelten Ultraschallsender auf das Zielvolumen unter einer orientierbaren Einstrahlrichtung gerichtet wird;
- mindestens ein ultraschall-basierter Schuss aus dem Ultraschallsender eingeleitet wird, welcher im Zielvolumen lokal und punktförmig auftrifft,
- mindestens eine Ultraschallsonde mindestens einen Anteil des Zielvolumen in einem Ultraschallbild aufnimmt,
- ein Mittel zur Ermittlung der Position einer ultraschall-basierten Markierung des Schusses in dem Bild verwendet wird,
- ein Abstand zwischen der Position der Markierung und einer Lage des zu kontrollierenden Auftreffens ermittelt und überprüft wird.

In anderen Worten ermöglicht das Verfahren, als Markierung ein lokales Verstärkungssichtsmittel an der Zielstelle des ersten Strahlsenders zu erzeugen. Diese lokale, sichtbare Verstärkung in dem Ultraschallbild wird als eine ultraschallselektive und punktförmige Wellenüberlagerung gebildet, deren Lage sehr genau ist und für den Patient nicht schädlich ist.

Die Strahlrichtung des Ultraschallsenders ist orientierbar, d.h. sie wird gewählt, um eine Zielstelle (= Isozentrum) des ersten Strahlsenders zu treffen und kann auch angepasst werden, um sich an der Zugänglichkeit des zutreffenden Zielvolumens bzw. des Körperteils (Lunge, Gehirn, Prostata, etc.) perfekt zu eignen. Unterschiedliche vordefinierte Strahlrichtungen können auch verwendet werden, um parallele oder serielle Schüsse zu erzeugen, so dass die Genauigkeit der Ermittlung des Abstandes räumlich eindeutig und verbessert wird oder/und so dass für die Ultraschallschüsse mögliche Schattenstellen des Gegenstandes oder des Patienten vermieden werden.

Eine sehr wichtige Konsequenz dieser Simulation liegt darin, dass ein Schuss aus dem ersten (hochenergetischen, d.h. schädlichen) Strahlsender als erlaubt definiert wird, nur wenn der ermittelte Abstand unter einer Toleranzschwelle liegt. Insbesondere bei einer gekoppelten Planung- oder Bestrahlungstherapie bildet also das erfindungsgemässe Verfahren eine hochqualitative Methode, um ungesunde Zellen exakt zu zerstören, ohne gesunde Zellen zu beschädigen.

Dadurch dass die Markierung eine Echtzeitpositionierung des Zielvolumens darstellt, kann der Gegenstand zwischen mehreren aufgenommenen Ultraschallbildern neu positioniert werden, so dass dabei ermittelte Abstände minimiert werden. In anderen Worten wird der Strahlsender bzw. mit oder ohne die Ultrasonde gegenüber dem Gegenstand bewegt, derart dass sich die Markierung auf einen im Ultraschallbild gewählten Punkt (z.B. das Zentrum eines Tumors) überlagert. Dies kann mittels einer motorischen Steuerung der Position des Strahlsenders entweder manuell von einem Operator oder automatisch mit Hilfe von Erkennungs- und Tracingsalgorithmen (welche die Markierung erkennen und die motorische Steuerung dementsprechend antreiben) gesteuert werden. Das Verfahren zur Strahlungssimulation ermöglicht daher eine Hilfe um eine manuelle Positionierung oder auch eine automatische Steuerung der Positionierung eines Patienten gegenüber dem ersten Strahlsender, wie einem Linearbeschleuniger.

Ebenfalls können der Ultraschallsender und die Ultraschallsonde zwischen sequentiell aufgenommenen Ultraschallbildern neu positioniert werden, so dass die dabei ermittelten Abstände minimiert werden. Dies kann ebenfalls durch eine Verwendung von mehreren Ultrasendern verbessert bzw. erleichtert werden. Wenn z.B. drei Ultraschallsender senkrechte Strahlungsachse aufweisen, welche einen einzigen Schnittpunkt bilden, ist es möglich, die drei damit überlagerten Ultraschall-Markierungen genauer an einer drei-dimensionalen Lagen eines Punkts im Zielvolumen zu setzen. Dabei werden die Abstände zwischen diesem Punkt und der drei-dimensionalen Markierung gemäß jedem der drei Strahlungsachse minimiert.

Ferner wird eine vorteilhafte Einrichtung zur Strahlungssimulation eines zu kontrollierenden Auftreffens eines ersten, zu richtenden Strahlsenders auf ein Zielvolumen eines Gegenstandes vorgeschlagen, welche folgende Elemente aufweist :
- mindestens einen dem ersten Strahlsender räumlich gekoppelten Ultraschallsender, welcher eine orientierbare Einstrahlrichtung auf das Zielvolumen und ein Mittel zur Einleitung eines ultraschall-basierten Schusses aufweist, wobei der Schuss im Zielvolumen lokal und punktförmig auftrifft;
- mindestens eine Ultraschallsonde, welche auf das Zielvolumen zur Aufnahme eines Ultraschallbildes sowie einer ultraschall-basierte Markierung aus mindestens einem Schuss gerichtet ist;

- erste Mitteln zur Ermittlung eines Abstandes zwischen der Markierung und des zu kontrollierenden Auftreffens im Zielvolumen;
- zweite Mitteln zur Kontrolle einer relativen Lage des Ultraschallsenders und der Ultraschallsonde gegenüber dem zu kontrollierenden Auftreffen.

Anschließend wird die Erfindung in Ausführungsbeispielen anhand der Zeichnungen erläutert.

Dabei zeigen
- FIG 1: eine erfindungsgemässe Einrichtung mit zwei Ultrastrahlsendern zur Strahlungssimulation ei- ner Bestrahlungsanlage,
- FIG 2: ein schematisches Beispiel zur Erläuterung der Verwendung der erfindungsgemässen Einrichtung für einen der Ultraschallsender,
- FIG 3: eine schematische Darstellung einer erfindungs- gemässen Strahlungssimulation und tomographi- schen Planungsbildern.

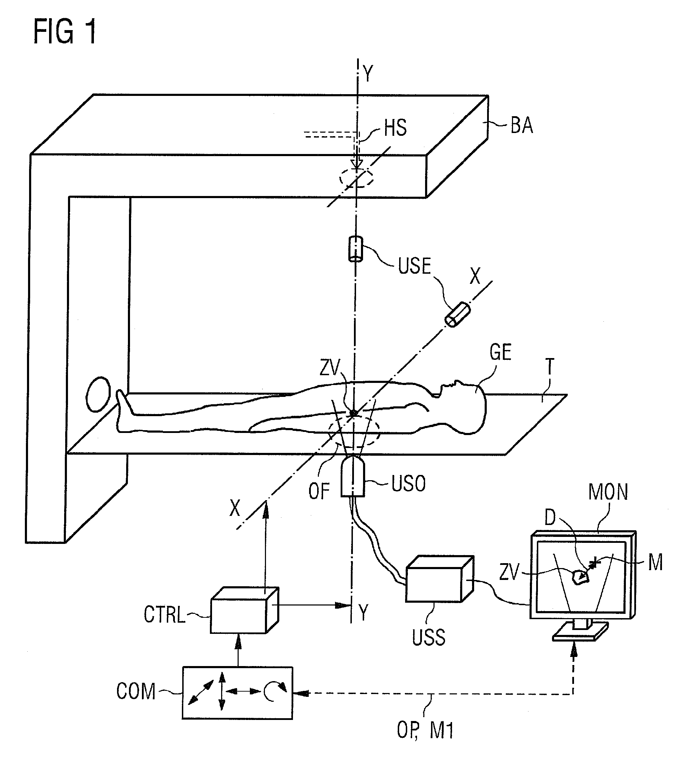

**Figur 1** stellt beispielsweise eine Bestrahlungsanlage BA (z.B. ein Linearbeschleuniger) mit einem ersten Strahlsender HS dar, welcher einen hochenergetischen Strahl zur Zerstörung eines Tumors in einem Zielvolumen eines Gegenstands (Phantoms) oder eines Patienten GE erzeugt. Das Zielvolumen ZV enthält auch ein sogenanntes Isozentrum der Bestrahlungsanlage, wobei die Energie fokussieren soll und sich konzentriert (insbesondere bei mehreren Strahlungsrichtungen). Der Patient GE liegt hier auf einem Tisch T, welcher räumlich und relativ gegenüber dem Isozentrum bewegt werden kann. An diesem Stadium ist der erste Strahlungssender ausgeschaltet und wird mittels mindestens eines anderen Ultraschallsenders USE ersetzt. Idealerweise kann der Ultraschallsender USE im gleichen Strahlweg Y-Y Ultraschallwellen verbreiten, jedoch ist es auch nicht zwingend, sobald die relative Lage zwischen dem ersten Strahlsender HS und dem Ultrasender USE bekannt ist.

Grundsächlich wird in Figur 1 eine mögliche Ausführung der erfindungsgemässen Einrichtung zur Strahlungssimulation eines zu kontrollierenden Auftreffens des ersten, zu richtenden Strahlsenders HS auf das Zielvolumen ZV des Gegenstandes GE gezeigt, welche folgende Elemente aufweist :
- mindestens (hier zwei) einen dem ersten Strahlsender räumlich gekoppelten Ultraschallsender USE, welcher eine orientierbare oder/und verschiebbare Einstrahlrichtung auf das Zielvolumen und ein Mittel zur Einleitung eines ultraschall-basierten Schusses aufweist, wobei der Schuss im Zielvolumen lokal und punktförmig auftrifft;
- die beiden Ultrasender USE weisen hier zwei senkrechte Strahlungsrichtungen oder -achse X-X, Y-Y auf, welche einen Schnittpunkt im Bereich des Zielvolumens ZV bilden; in diesem Ausführungsbeispiel stimmt die hier vertikale Strahlungsachse (Y-Y) einfach mit der Strahlungsachse der zu simulierten Bestrahlungsanlage überein, um einen Rechenaufwand von relativen Koordinaten zu vermeiden (wie oben bereits erwähnt); die andere, hier horizontale Strahlungsachse X-X des zweiten Ultraschallsenders USE ist parallel zu einer transversalen Achse der Tischlänge, so dass sie das Zielvolumen durchqueren kann;
- mindestens eine Ultraschallsonde USO, welche auf das Zielvolumen zur Aufnahme eines Ultraschallbildes USB sowie einer ultraschall-basierte Markierung M aus mindestens einem Schuss des oder beiden Ultrasenders USE gerichtet ist;
- erste Mitteln M1 zur Ermittlung eines Abstandes D zwischen der Markierung und des zu kontrollierenden Auftreffens im Zielvolumen; diese ersten Mitteln M1 werden aus einer adaptiven Monitoringstelle MON angeschlossen, welche über eine Schnittstelle USS am Ausgang der Ultraschallsonde USO nachgeschaltet ist; An der Monitoringstelle ist es möglich, einerseits das aufgenommene Ultraschallbild der Ultraschallsonde (mit dem Zielvolumen ZV) und andererseits die übergelagerte Markierung M zu beobachten; In Figur 1 ist eine leichte Abweichung zwischen dem Zentrum des Zielvolumens und der Markierung, welche während einer Strahlungssimulation genau der zu minimierende Abstand D ist;
- zweite Mitteln COM, CTRL zur Kontrolle und Steuerung einer relativen Lage und ggf. Orientierung des/der Ultraschallsender(s) USE und der Ultraschallsonde USO gegenüber dem zu kontrollierenden Auftreffen (hier auf einen Tumor im Zielvolumen); diese zweite Mitteln können von einem Operator OP oder von automatischen Erkennungs- und Tracing-Tools M1 algorithmisch an einem Rechner in Verbindung mit der Ultraschallsonde USO ausgeführt werden.

Die dargestellte Einrichtung sieht vor, dass mindestens der Achse Y-Y des vertikalen Ultraschallsenders USO im Sichtfeld der Ultraschallsonde USO sich befindet bzw. bewegbar ist. Bei mehreren Ultraschallsendern mit geneigten Strahlungsachsen wie in Figur 1 X-X, Y-Y wesentlich ist es, dass der Schnittpunkt aller Achse im Sichtfeld der Ultraschallsonde sich befindet bzw. bewegbar ist, damit die Markierung an einem Ort des Ultraschallbildes aus der Ultraschallsonde aufgenommen wird.

In Figur 1 liegt der Gegenstand GE (z.B. ein Phantom oder ein Patient) zwischen dem oberen Ultraschallsender USE mit vertikalen Achse Y-Y und der unteren Ultraschallsonde USO. Damit die Ultraschallsonde eine Aufnahme eines Bereiches des Gegenstandes machen kann, ist eine Öffnung OF in dem Tisch T zum Durchlass von Ultraschallwellen über den Tisch vorgesehen. Der Ultraschallsender kann mit dem Gegenstand kontaktlos positioniert werden. Jedoch ist die Ultraschallsonde USO gegen eine Fläche des Gegenstandes (hier am Rücken des Patienten) angebracht, hilfsweise mittels eines zwischen liegenden akustischen Gels.

Die Einrichtung kann so gebildet werden, dass mindestens der Ultraschallsender und die Ultraschallsonde eine synchrone Bewegung relativ zu dem Gegenstand GE (bzw. dem Zielvolumen) aufweisen, welche zumindest in Ebenen senkrecht zur Einstrahlungsachse des hier vertikalen Ultraschallsenders bzw. zu einer Hauptachse der Ultraschallsonde (d.h. zu der senkrechten Achse zur Bildmitte seiner Aufnahme) vorliegt. Damit kann das Zielvolumen einfach verfolgt werden, insbesondere wenn der Gegenstand lebendig ist und sich selber bewegen kann.

Damit eine Bewegung der Ultraschallsonde relativ zum Gegenstand einwandfrei erfolgen kann, d.h. dass die Kanalisierung der Ultraschallwellen immer gleich gut geschehen kann, kann eine gleitende Kontaktfläche oder -volumen vorgesehen werden, so dass sie einen akustischen Kanal zwischen der Ultraschallsonde und dem Gegenstand bildet. Dies kann unter der Form eines mit akustischem Gel füllbaren Kissens zwischen dem Gegenstand und der Ultraschallsonde realisiert werden.

Die erfindungsgemässe Einrichtung kann mehrere Ultraschallsender aufweisen, wie in Figur 1, wobei zwei Ultraschallsender winkelgeneigte, hier senkrechte Einstrahlungsachse aufweisen, deren Schnittpunkt im Sichtfeld der Ultraschallsonde bewegbar ist. Es können noch mehr als zwei Ultraschallsender angeordnet werden, deren alle Strahlungsachse auf einen einzelnen Schnittpunkt zur besseren Bildung der Markierung gerichtet sind. Damit können mögliche Schattenstellen für Ultraschallwellen während einer Bewegung des Gegenstands, oder des Zielvolumens gegenüber einem Komponente der Einrichtung vermieden werden bzw. ein höheres Signal-Rausch-Verhältnis der zu detektierenden Markierung im Ultraschallbild erzielt werden.

Eine weitere Alternative zur Verbesserung der aufgenommenen Abbildung bzw. der Detektion des Zielvolumens kann auch erreicht werden, indem das Zielvolumen ein ultraschall-basiertes Kontrastmittel aufweist. Dieses Kontrastmittel ist an das Zielvolumen vor der Aufnahme mit der Ultraschallsonde angebracht, und wirkt während der Aufnahme sowie einer weiteren Behandlung.

Schließlich sind es, verschiedene, vorteilhafte Verwendungen der erfindungsgemässen Einrichtung zu nennen.

Eine Verwendung der Einrichtung dient zur Vorpositionierung eines Strahlungsauftreffpunktes einer Bestrahlungsanlage BA wie des Linearbeschleunigers aus Figur 1 gegenüber einem zu strahlenden Bereich des Zielvolumens ZV, mit Berücksichtigung von räumlichen Offsetwerten zur Lage des Bereiches.

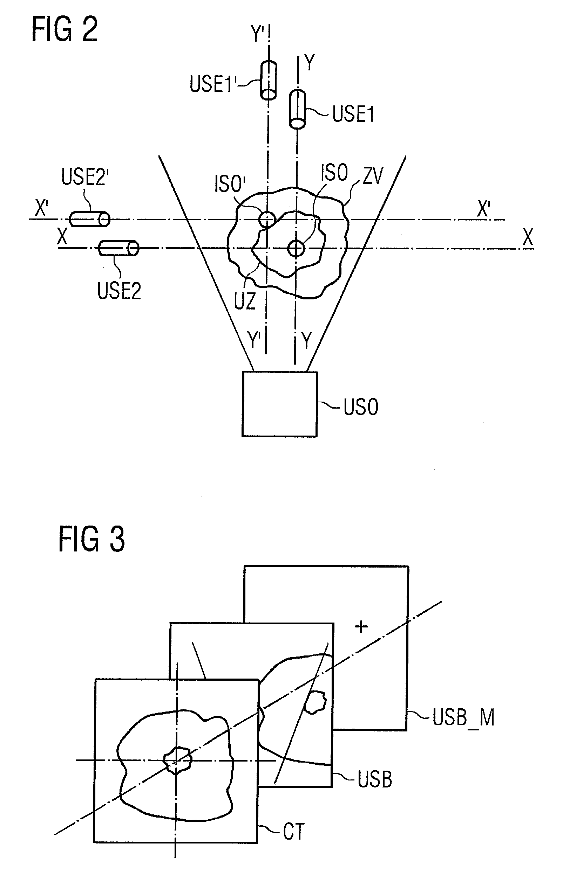

**Figur 2** illustriert diesen letzten Punkt besser, indem eine Vorpositionierung eines Isozentrums ISO' auf einer ultraschall-basierten Schnittaufnahme X-X, Y-Y des Inneren des Gegenstandes ermöglicht ist. Dieses Isozentrum ISO' ist einfach durch die ultraschall-basierte Markierung gebildet und ersetzt das virtuelle Isozentrum der ausgeschalteten Bestrahlungsanlage. Dabei werden der Ultraschallsender und bei Bedarf auch die Ultraschallsonde relativ zum Gegenstand gefahren, derart dass die als virtuelle Isozentrum dienende Markierung ISO' auf ein gezieltes Punkt ISO des Zielvolumens des Gegenstandes überlagert wird, mit Berücksichtigung von räumlichen Offsetwerten zur Lage des gezielten Punktes. Das gezielte Punkt ISO bildet daher das tatsächliche, reale Isozentrum, auf welches die Bestrahlungsanlage Ihre Energie konzentrieren kann.

Angenommen dass das zu kontrollierende Auftreffen ein vordefiniertes Isozentrum des zu steuernden Strahlensenders bildet, ist es mittels der erfindungsgemässen Einrichtung aus Figur 1 möglich, das zu kontrollierende Auftreffen eines z.B. falsch zielendes, vordefinierten Isozentrums ISO' eines dazugehörigen Strahlengerätes genau auf ein nun (richtiges) sichtbares, markiertes Isozentrum ISO am Zielvolumen zu positionieren.

Ferner ist eine weitere Verwendung der erfindungsgemässen Einrichtung in **Figur 3** gezeigt, wobei das Ultraschallbild (hier mit zwei Ultraschallebenen USB, USB_M dargestellt, zur schematischen Trennung der Aufnahme des Zielvolumens und der Markierung) auf einer davor durchgeführten tomographischen Schnittaufnahme CT mittels teildurchsichtigen Ansichten überlagert wird. Damit ist eine vorteilhafte Fusion von Daten aus einer erfindungsgemässen Strahlungssimulation und aus tomographischen Planungsbildern ermöglicht.

Alle Verwendungen der erfindungsgemässen Einrichtung aus Figuren 1 bis 3 sind insbesondere in Sinnen von Detektionsempfindlichkeit perfekt geeignet, wenn das Zielvolumen ungesunde Zellen UZ wie Tumorzellen aufweist. Derartigen Ultraschalltechniken sind ebenfalls für Patienten unschädlich. Dadurch dass auch die Methode und Einrichtung zur Strahlungssimulation sehr genau auf ungesunde Zellen gerichtet sind, werden gesunde Nachbarnzellen unbeschädigt werden, wenn z.B. eine nachträgliche, radiotherapeutische Behandlung stattfinden sollte.

## Patentansprüche

1. Verfahren zur Strahlungssimulation eines zu kontrollierenden Auftreffens eines ersten, zu richtenden Strahlsenders (HS) auf ein Zielvolumen (ZV) eines Gegenstandes (GE),
**dadurch gekennzeichnet, dass:**
- mindestens ein dem ersten, stillgelegten Strahlsender (HS) räumlich gekoppelten Ultraschallsender (USE) auf das Zielvolumen unter einer orientierbaren Einstrahlrichtung (XX, YY) gerichtet wird;
- mindestens ein ultraschall-basierter Schuss aus dem Ultraschallsender eingeleitet wird, welcher im Zielvolumen lokal und punktförmig auftrifft,
- mindestens eine Ultraschallsonde (USO) mindestens einen Anteil des Zielvolumen in einem Ultraschallbild (USB) aufnimmt,
- ein Mittel zur Ermittlung der Position einer ultraschall-basierten Markierung (M) des Schusses in dem Bild verwendet wird,
- ein Abstand (D) zwischen der Position der Markierung und einer Lage des zu kontrollierenden Auftreffens ermittelt und überprüft wird.

2. Verfahren nach Anspruch 1, **dadurch gekennzeichnet, dass** ein Schuss aus dem ersten Strahlsender als erlaubt definiert wird, nur wenn der Abstand unter einer Toleranzschwelle liegt.

3. Verfahren nach Anspruch 1 oder 2, **dadurch gekennzeichnet, dass** der Gegenstand zwischen aufgenommenen Ultraschallbildern neu positioniert wird, so dass dabei ermittelte Abstände minimiert werden.

4. Verfahren nach Anspruch 1 oder 2, **dadurch gekennzeichnet, dass** der Ultraschallsender und die Ultraschallsonde zwischen aufgenommenen Ultraschallbildern neu positioniert werden, so dass dabei ermittelte Abstände minimiert werden.

5. Einrichtung zur Strahlungssimulation eines zu kontrollierenden Auftreffens eines ersten, zu richtenden Strahlsenders (HS) auf ein Zielvolumen eines Gegenstandes, **gekennzeichnet durch:**
- mindestens einen dem ersten Strahlsender räumlich gekoppelten Ultraschallsender (USE), welcher eine orientierbare Einstrahlrichtung auf das Zielvolumen und ein Mittel zur Einleitung eines ultraschall-basierten Schusses aufweist, wobei der Schuss im Zielvolumen lokal und punktförmig auftrifft;
- mindestens eine Ultraschallsonde (USO), welche auf das Zielvolumen zur Aufnahme eines Ultraschallbildes (USB) sowie einer ultraschall-basierte Markierung (M) aus mindestens einem Schuss gerichtet ist;
- erste Mitteln (M1) zur Ermittlung eines Abstandes (D) zwischen der Markierung und des zu kontrollierenden Auftreffens im Zielvolumen;
- zweite Mitteln (COM, CTRL) zur Kontrolle einer relativen Lage des Ultraschallsenders und der Ultraschallsonde gegenüber dem zu kontrollierenden Auftreffen.

6. Einrichtung nach Anspruch 5, wobei das zu kontrollierende Auftreffen ein vordefiniertes Isozentrum (ISO, ISO') des zu steuernden Strahlensenders bildet.

7. Einrichtung nach Anspruch 5 oder 6, wobei der Achse des Ultraschallsenders im Sichtfeld der Ultraschallsonde bewegbar ist.

8. Einrichtung nach Anspruch 7, wobei der Gegenstand zwischen dem Ultraschallsender und der Ultraschallsonde liegt.

9. Einrichtung nach Anspruch 8, wobei der Ultraschallsender und die Ultraschallsonde eine synchrone Bewegung aufweisen, welche zumindest in Ebenen senkrecht zur Einstrahlungsachse vorliegt.

10. Einrichtung nach Anspruch 9, wobei eine gleitende Kontaktfläche oder -volumen einen akustischen Kanal zwischen der Ultraschallsonde und dem Gegenstand bildet.

11. Einrichtung nach einem der vorigen Ansprüche, wobei zwei Ultraschallsender winkelgeneigte, wie senkrechte Einstrahlungsachse aufweisen, deren Schnittpunkt im Sichtfeld der Ultraschallsonde bewegbar ist.

12. Einrichtung nach einem der vorigen Ansprüche, wobei das Zielvolumen ein ultraschall-basiertes Kontrastmittel aufweist.

13. Verwendung der Einrichtung gemäß Ansprüchen 5-12 zur Vorpositionierung eines Strahlungsauftreffpunktes einer Bestrahlungsanlage (BA) gegenüber einem zu strahlenden Bereich des Zielvolumens, mit Berücksichtigung von räumlichen Offsetwerten zur Lage des Bereiches.

14. Verwendung der Einrichtung gemäß Ansprüchen 5-12 zur Vorpositionierung eines Isozentrums auf einer Schnittaufnahme des Inneren des Gegenstandes, wobei der Ultraschallsender und bei Bedarf auch die Ultraschallsonde relativ zum Gegenstand gefahren werden, derart dass die als Isozentrum dienende Markierung auf ein gezieltes Punkt des Zielvolumens des Gegenstandes überlagert wird, mit Berücksichtigung von räumlichen Offsetwerten zur Lage des gezielten Punktes.

15. Verwendung nach Anspruch 14, wobei das Ultraschallbild (USB, USB_M) auf die Schnittaufnahme (CT) mittels teildurchsichtigen Ansichten überlagert wird.

16. Verwendung nach einem der Ansprüche 13 bis 15, wobei das Zielvolumen ungesunde Zellen (UZ) wie Tumorzellen aufweist.
